# EUROPÄISCHE PATENTANMELDUNG

(11) **EP 4 840 484 A1**
(43) Veröffentlichungstag der Anmeldung: **18.11.2026**
(21) Anmeldenummer: 25177075.6
(22) Anmeldetag: 16.05.2025
(51) Int. Cl.: A61M 1/36, A61M 5/36, G01N 29/032

(54) **VERFAHREN UND VORRICHTUNG ZUR ADAPTIVEN DETEKTION VON GASBLASEN IN EINEM INFUSIONSSCHLAUCH ODER BLUTSCHLAUCH MITTELS ULTRASCHALL**

(71) Anmelder: B. Braun Melsungen AG, 34212 Melsungen (DE)
(72) Erfinder: KRAPF, Johannes, 36179 Bebra (DE); HOLLSTEIN, Rolf, 36211 Alheim-Heinebach (DE); BRESSAU, Ernst, 34323 Malsfeld (DE)
(74) Vertreter: Tergau & Walkenhorst Intellectual Property GmbH

(57) **Zusammenfassung**

Ein Verfahren zur Detektion von Gasblasen, insbesondere Luftblasen, in einem von einem Infusionsmedium durchströmten Infusionsschlauch (4) oder in einem von Blut durchströmten Blutschlauch (10) eines Dialysesystems, bei dem ein ultraschallbasierter Gassensor (1) eingesetzt wird, soll eine zuverlässige Detektion und gegebenenfalls auch Bilanzierung von Gasblasen im Infusionsschlauch (4) oder Blutschlauch (10) ermöglichen, auch unter Bedingungen niedriger Flussraten oder bei im Sensorbereich temporär festgesetzten Gasblasen. Erfindungsgemäß umfasst das Verfahren die Schritte:
• Applizieren von Ultraschallimpulsen auf den Infusionsschlauch (4) oder Blutschlauch (10) mittels eines Ultraschallsenders (2),
• Detektion von durch den Infusionsschlauch (4) oder Blutschlauch (10) propagierten Ultraschallimpulsen durch einen Ultraschallempfänger (3),
• Auswertung eines vom Ultraschallempfänger (3) ausgegebenen Sensorsignals durch eine Steuer- und Auswerteeinheit (8),
• Vergleich des Sensorsignals oder eines daraus abgeleiteten Signals mit einem vorgegebenen Schwellenwert zur Erkennung von Gasblasen,
• Ausgabe eines Alarmsignals, wenn das Sensorsignal den Schwellenwert überschreitet,
wobei während der Auswertung mindestens einer der folgenden Parameter automatisiert angepasst oder variiert wird:
• die mittels der Ultraschallimpulse in den Infusionsschlauch (4) oder Blutschlauch (10) eingebrachte Sendeenergie,
• der Schwellwert zur Auslösung des Alarmsignals.

## Beschreibung

### Technisches Gebiet

Die vorliegende Erfindung betrifft das technische Gebiet der medizinischen Gerätetechnik, insbesondere Systeme zur Überwachung von Flüssigkeitsströmen in Infusionsschläuchen von Infusionsgeräten oder in Blutschläuchen von Dialysegeräten. Die Erfindung betrifft ein Verfahren sowie eine Vorrichtung zur Detektion und gegebenenfalls Bilanzierung von Gasblasen, insbesondere Luftblasen, innerhalb eines von einem Infusionsmedium durchströmten Infusionsschlauchs oder eines von Blut durchströmten Blutschlauchs unter Verwendung eines Ultraschallsensorsystems. Die Erfindung ist insbesondere für den Einsatz in Infusions- und Dialysesystemen sowie extrakorporalen Blutkreisläufen geeignet.

### Stand der Technik

Im Stand der Technik ist die Detektion von Luft oder anderen Gasen in Flüssigkeitsschläuchen, insbesondere in Infusionsschläuchen, häufig über Ultraschallsensoren realisiert. Dabei werden Ultraschallimpulse durch den Schlauch geschickt, und die Veränderung der Signaldämpfung bzw. Signalstreuung durch im Medium enthaltene Gasblasen ausgewertet. Ein Vergleich mit einem festen Schwellenwert entscheidet über das Vorhandensein von Gaseinschlüssen, insbesondere Luftblasen.

Insbesondere bei sehr niedrigen Flussraten, wie sie in Infusionssystemen häufig auftreten, können sich kleine Gasblasen jedoch im Sensorbereich festsetzen oder langsam zu größeren Blasen aggregieren. Dieses Verhalten hängt stark von Schlauchlage, Schlauchmaterial und Sensorgeometrie ab. Zusätzlich unterliegen der Infusionsschlauch und die darin befindliche Infusionslösung häufig physikalischen Schwankungen wie beispielsweise der Temperatur, welche das Sensorsignal beeinflussen. Die festen Schwellenwerte des Stands der Technik führen unter diesen Bedingungen zu unerwünschten Systemreaktionen.

### Aufgabe der Erfindung

Es ist daher die Aufgabe der vorliegenden Erfindung, ein Verfahren sowie eine Vorrichtung bereitzustellen, die eine zuverlässige Detektion und gegebenenfalls auch Bilanzierung von Gasblasen in einem Infusionsschlauch oder Blutschlauch ermöglichen, auch unter Bedingungen niedriger Flussraten oder bei im Sensorbereich temporär festgesetzten Gasblasen. Ziel ist insbesondere, die Abhängigkeit der Gasdetektion von der dynamischen Position einzelner Gasblasen zu reduzieren.

### Lösung der Aufgabe

Diese Aufgabe wird erfindungsgemäß durch ein Verfahren mit den Merkmalen des Anspruchs 1 sowie durch eine Vorrichtung mit den Merkmalen des Anspruchs 13 gelöst.

Demnach wird ein ultraschallbasiertes Verfahren zur Detektion von Gasblasen in einem Infusionsschlauch oder Blutschlauch bereitgestellt. Dabei wird ein Sensorsignal, das durch einen Ultraschallempfänger erzeugt wird, in einer Steuer- und Auswerteeinheit ausgewertet und mit einem Schwellenwert verglichen. Ein Alarmsignal wird ausgegeben, wenn das Sensorsignal den Schwellenwert über- oder unterschreitet. Wesentlich ist dabei, dass während der Auswertung mindestens einer der folgenden Parameter automatisiert angepasst oder variiert wird: (i) die in den Schlauch eingebrachte Sendeenergie der Ultraschallimpulse und/oder (ii) der Schwellenwert zur Auslösung des Alarms.

Die Regelung dieser Parameter erlaubt eine dynamische Adaption an wechselnde physikalische Gegebenheiten wie Medienart, Temperatur, Schlauchmaterial, Flussverhalten und Luftverteilung. So kann die Erkennung auch unter herausfordernden Bedingungen stabil erfolgen. Das Verfahren ist dabei insbesondere in der Lage, sogenannte "stehende Luftblasen", also über längere Zeiträume im Sensorbereich verweilende Gasblasen, zu tolerieren und bei deren Weitertransport korrekt zu bilanzieren.

Des Weiteren wird eine Vorrichtung bereitgestellt, die unter anderem aus einem Ultraschallsender, einem Ultraschallempfänger, einer Steuer- und Auswerteeinheit, einer Vergleichseinheit sowie einer Alarmeinheit besteht. Eine Steuerungs- oder Regelungseinheit ist zusätzlich vorgesehen, um mindestens einen der oben genannten Parameter - Sendeenergie oder Alarmschwelle - automatisiert anzupassen. Die Vorrichtung ist damit technisch ausgestaltet, das erfindungsgemäße Verfahren auszuführen.

Die Vorteile und Merkmale der beschriebenen Ausgestaltungen des Verfahrens lassen sich in entsprechender Weise auch auf die Vorrichtung übertragen. Umgekehrt gilt, dass die technischen Ausgestaltungen der Vorrichtung insbesondere der Durchführung und Optimierung des Verfahrens dienen. Soweit Merkmale als verfahrensspezifisch beschrieben sind, gelten sie daher sinngemäß auch für die entsprechende Vorrichtung und umgekehrt.

### Vorteilhafte Ausgestaltungen

In bevorzugter Ausgestaltung erfolgt die Anpassung der Sendeenergie durch eine Steuerung der Anzahl aufeinanderfolgender Ultraschallimpulse und/oder durch Regelung des Energieinhalts einzelner Impulse. Diese Maßnahme erlaubt eine besonders flexible Abstimmung der Energieeinbringung auf unterschiedliche Schlauch- und Flüssigkeitseigenschaften.

In bevorzugter Ausgestaltung kann der Energieinhalt einzelner Ultraschallimpulse durch die Steuerung der Ladezeit einer im Sendekreis des Ultraschallsenders angeordneten Induktivität beeinflusst werden. Durch Variation der Ladezeit wird die in der Induktivität gespeicherte Energie geregelt, die anschließend während der Entladung zur Ansteuerung des Ultraschallsenders verwendet wird. Je länger die Ladezeit (bei konstanter Versorgungsspannung), desto größer ist die im Magnetfeld der Induktivität gespeicherte Energie, und desto energiereicher fällt der erzeugte Ultraschallimpuls aus. Dies erlaubt eine feindosierte Regelung der eingekoppelten Ultraschallenergie in Abhängigkeit von äußeren Bedingungen wie Schlauchmaterial, Füllmedium oder Gasgehalt. Die Verwendung einer steuerbaren Ladezeit zur Beeinflussung des Energieinhalts stellt eine besonders einfache und robuste Möglichkeit zur dynamischen Leistungsanpassung im laufenden Betrieb dar, ohne dass eine aufwendige analoge Leistungsregelung erforderlich wäre.

Vorteilhafterweise wird der Schwellenwert zur Auslösung des Alarmsignals und/oder die Sendeenergie dynamisch in Abhängigkeit vom Verlauf des Sensorsignals über die Zeit angepasst.

**In** bevorzugter Ausgestaltung erfolgt die Regelung der Alarmschwelle und/oder der Sendeenergie anhand eines vorgegebenen Zielbereichs für das Sensorsignal, in dem die Unterscheidung zwischen Gasblasen und Flüssigkeit zuverlässig möglich ist. Durch den Vergleich aktueller Messwerte mit dem Zielbereich kann eine automatische Nachregelung der Sendeenergie erfolgen, was zu einem stabileren Sensorbetrieb führt.

Vorteilhafterweise wird die automatisierte Anpassung durch einen in der Steuer- und Auswerteeinheit implementierten Algorithmus gesteuert, der charakteristische Signalverläufe analysiert und unterschiedlichen physikalischen Situationen zuordnet, insbesondere stehenden oder bewegten Gasblasen.

Vorteilhaft ist eine Version der Verfahrens, bei der über ein festgelegtes Zeitintervall Mittelwerte oder andere statistische Größen des Sensorsignals gebildet werden und die entsprechenden Messwerte verworfen und eine neue Mittelwertbildung gestartet wird, wenn die maximale Differenz innerhalb der Messwerte einen festgelegten Schwellenwert unter- oder überschreitet.

**In** bevorzugter Ausgestaltung basiert der Algorithmus auf einer Lookup-Tabelle oder einem lernfähigen Modell, das im Vorfeld oder im laufenden Betrieb trainiert wurde. Dies erlaubt eine adaptive Anpassung auf spezifische Gerätekonfigurationen oder Patientenbedingungen.

Vorteilhafterweise wird zusätzlich zur Detektion auch eine Bilanzierung des Gasvolumens vorgenommen. Hierzu wird das Verlassen des Sensorbereichs durch Gasblasen anhand von Signalverlauf und -änderung erkannt und quantifiziert. Dies kann insbesondere dadurch erfolgen, dass beim plötzlichen Anstieg des Sensorsignals infolge des Verlassens einer im Sensorbereich stehenden Gasblase ein Vorher-Nachher-Vergleich durchgeführt wird, um das Verschwinden der Blase zu erkennen und deren Volumen näherungsweise zu bestimmen.

In bevorzugter Ausgestaltung wird ein Alarm nur dann ausgelöst, wenn die Überschreitung des Schwellenwerts über eine definierte Mindestzeit andauert oder die Flankensteilheit des Sensorsignals eine bestimmte Grenze überschreitet. Diese Maßnahme dient zur Vermeidung von unerwünschten Alarmen bei kurzen Signalabweichungen.

In bevorzugter Ausgestaltung erfolgt eine Plausibilitätsprüfung zur Unterscheidung echter Gasblasen von Signalartefakten, etwa durch Umwelteinflüsse.

Vorteilhafterweise wird die Auswertung abhängig vom Betriebsmodus der Infusionseinrichtung durchgeführt. Dabei können z. B. im Vorbereitungsmodus andere Auswertealgorithmen als im Therapiebetrieb angewendet werden.

In bevorzugter Ausgestaltung wird die Steuerung zusätzlich durch weitere Eingangsinformationen gespeist, wie etwa die Art des Infusionsmediums, die aktuelle Flussrate oder die geometrische Anordnung des Schlauchs im Sensor. Diese Daten können in die Steuer- oder Regelstrategie einfließen, um die Detektionsgenauigkeit weiter zu erhöhen.

In einer bevorzugten Ausgestaltung kann die Steuer- und Auswerteeinheit vollständig in den Gassensor integriert sein. Dadurch entsteht eine besonders kompakte und platzsparende Bauform, bei der sämtliche Signalverarbeitungsschritte einschließlich Schwellenwertvergleich, Regelung der Sendeenergie und Alarmauslösung innerhalb des Sensorgehäuses erfolgen. Alternativ kann die Steuer- und Auswerteeinheit auch teilweise oder vollständig als separate Komponente ausgeführt sein, etwa in Form eines externen Steuergeräts oder als Bestandteil eines übergeordneten Infusions- oder Dialysesystems und/oder als Cloudservice. Eine solche Auslagerung kann insbesondere dann vorteilhaft sein, wenn komplexere Signalverarbeitung oder Kommunikation mit weiteren Systemkomponenten erforderlich ist.

Der Gassensor kann als Transmissions- oder als Reflexionsstrecke ausgebildet sein. Bei einer Transmissionsanordnung sind Ultraschallsender und -empfänger auf gegenüberliegenden Seiten des Infusionsschlauchs angeordnet, sodass die Ultraschallimpulse den Schlauch vollständig durchqueren. Diese Anordnung ermöglicht eine direkte Erfassung der Signalabschwächung durch im Schlauch befindliche Gasblasen. Alternativ kann der Gassensor als Reflexionssystem ausgeführt sein, bei dem Sender und Empfänger auf derselben Seite des Schlauchs in Form einer Ultraschallsende- und Empfangseinheit angeordnet sind und ein Ultraschallreflektor gegenüberliegend angebracht ist. In dieser Konfiguration wird das ausgesendete Ultraschallsignal am Ultraschallreflektor zurückgeworfen und vom Empfänger detektiert. Die Reflexionsanordnung erlaubt eine besonders kompakte Bauweise und kann beispielsweise dann vorteilhaft sein, wenn der Zugang zu nur einer Seite des Schlauchs möglich ist. Beide Varianten eignen sich gleichermaßen zur Durchführung des erfindungsgemäßen Verfahrens. Zusätzlich wird die durch die Verdopplung der Wegstrecke, die das ausgesendete Ultraschallsignal zurücklegt, die Messgenauigkeit des Verfahrens verbessert.

Die bislang im Zusammenhang mit Infusionssystemen beschriebenen Merkmale, Vorteile und Anwendungsbereiche der Erfindung gelten in gleicher Weise auch für Blutleitungssysteme von Dialysegeräten. Der Blutschlauch eines Dialysegerätes unterlegt in technischer und funktionaler Hinsicht vergleichbaren Anforderungen hinsichtlich der Gasblasenüberwachung wie ein Infusionsschlauch.

Insbesondere bei Hämodialyseverfahren ist die sichere und gasblasenfreie Verbindung zwischen dem extrakorporalen Kreislauf und dem Patienten von zentraler Bedeutung. Eine Vorrichtung, wie sie in der vorliegenden Erfindung beschrieben ist, kann auch hier eine erhöhte Betriebssicherheit gewährleisten, indem sie eine zuverlässige Detektion und ggf. Bilanzierung von Gasblasen oder eine automatische Alarmierung bei Abweichungen ermöglicht. Die erfindungsgemäßen Prinzipien lassen sich daher direkt auf den Blutschlauch eines Dialysegerätes übertragen, ohne dass wesentliche technische Modifikationen erforderlich wären. Der Schutzbereich dieser Anmeldung erstreckt sich somit ausdrücklich auch auf Anwendungen im Bereich der Dialysetechnik.

Der Begriff "Schlauch" kann in dieser Anmeldung bevorzugt einen flexiblen Schlauch, alternativ aber auch eine vergleichsweise starre Leitung, ein Rohr oder einen Strömungskanal umfassen, sofern das beschriebene Ultraschallmessverfahren damit kompatibel ist.

### Zusammenfassung

Die Erfindung betrifft zusammengefasst ein Verfahren und eine Vorrichtung zur ultraschallbasierten Detektion und optionalen Bilanzierung von Gasblasen in einem Infusionsschlauch oder Blutschlauch. Durch die automatisierte Steuerung oder Regelung der Sendeenergie und/oder der Alarmschwelle in Abhängigkeit von Sensorsignalen wird eine robuste, fehlertolerante und adaptiv einstellbare Luftdetektion ermöglicht. Die Erfindung eignet sich insbesondere zur Anwendung in medizinischen Infusionssystemen mit niedrigen Flussraten, in denen klassische Detektionssysteme häufig zu unerwünschten Alarmen oder Systemreaktionen führen.

### Kurzzusammenfassung der Figuren

**Fig. 1** zeigt im Querschnitt einen Infusionsschlauch mit einem zugeordneten Gassensor, verwirklicht als Transmissions-Ultraschallstrecke mit gegenüberliegendem Sender und Empfänger.
**Fig. 2** zeigt eine Variation der Anordnung aus Fig. 1, umfassend eine Reflexions-Ultraschallstrecke mit einem Reflektor und einem gemeinsamen Sender-Empfänger-Element auf einer Seite des Infusionsschlauchs.
**Fig. 3** zeigt ein Blockdiagramm eines Systems zur Detektion von Gasblasen in einem Infusionsschlauch, basierend auf einer Anordnung gemäß Fig. 1 oder Fig. 2, mit einer Anzahl von Komponenten zur Regelung der Sendeenergie durch Variation der Ladezeit und/oder der Anzahl von Sendeimpulsen.
**Fig. 4** zeigt ein Blockdiagramm eines entsprechenden Systems mit Komponenten zur Anpassung der Alarmschwelle in Abhängigkeit vom Sensorsignal.
**Fig. 5** zeigt den zeitlichen Verlauf eines mittels eines Gassensors erfassten Dämpfungssignals im Sensorbereich mit Beispielen für unterschiedliche Akkumulations- und Ablöseprozesse von Luftblasen.

### Detaillierte Beschreibung eines Ausführungsbeispiels

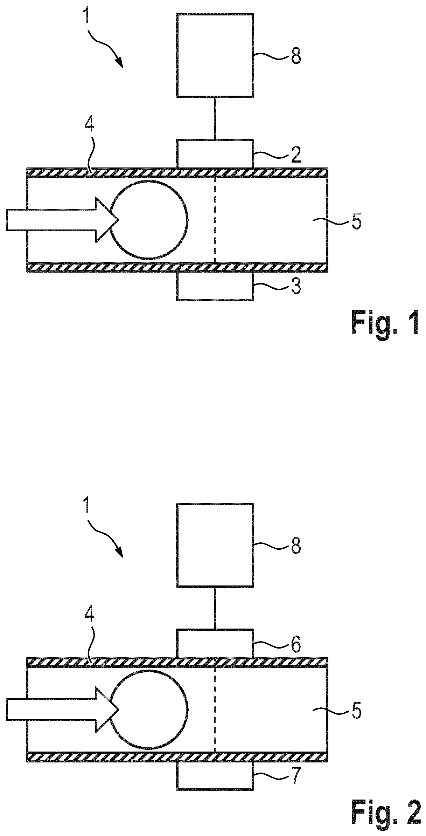

Fig. 1 zeigt ein bevorzugtes, erfindungsgemäßes Ausführungsbeispiel des Gassensors 1 und dessen Anordnung um den Infusionsschlauch 4, in dem Gasblasen detektiert werden sollen. Dabei handelt es sich um eine Transmissionsstrecke, in der Ultraschallsender 2 und Ultraschallempfänger 3 beidseitig gegenüber dem Infusionsschlauch 4 angeordnet sind. Der Infusionsschlauch 4 wird vom Ultraschallsender 2 und vom Ultraschallempfänger 3 umschlossen. Der Bereich des Infusionsschlauchs 4, der vom Gassensor 1 erfasst wird, ist der Sensorbereich 5 zwischen Ultraschallsender 2 und Ultraschallempfänger 3. Diese Anordnung ermöglicht eine Fixierung des Infusionsschlauchs 4 und insbesondere des Sensorbereichs 5, wodurch Messfehler minimiert werden. Zugleich wird durch die gegenüberliegende Positionierung von Ultraschallsender 2 und Ultraschallempfänger 3 der gesamte Querschnitt des Infusionsschlauchs 4 während der Messung erfasst.

Ein weiteres bevorzugtes und erfindungsgemäßes Ausführungsbeispiel des Gassensors 1 ist in Fig. 2 gezeigt. In dieser Ausführungsform sind Ultraschallsender 2 und Ultraschallempfänger 3 in einer Ultraschallsende- und Empfangseinheit 6 in Form von einer Komponente zusammengefasst. Dies hat den besonderen Vorteil, dass hierdurch die Anzahl elektrischer Bauteile verringert und eine kompaktere Bauweise ermöglicht wird. Der Infusionsschlauch 4 wird in diesem Ausführungsbeispiel von der Ultraschallsende- und Empfangseinheit 6 und zusätzlich einem Ultraschallreflektor 7 umschlossen, welcher gegenüber der Ultraschallsende- und Empfangseinheit positioniert ist. Diese Variante hat ebenfalls den Vorteil, dass die Position von Infusionsschlauch 4 und Sensorbereich 5 gesichert wird. Zusätzlich passieren in dieser Ausführungsvariante die Ultraschallimpulse den Sensorbereich 5 ein weiteres Mal, wodurch die Messgenauigkeit erhöht wird.

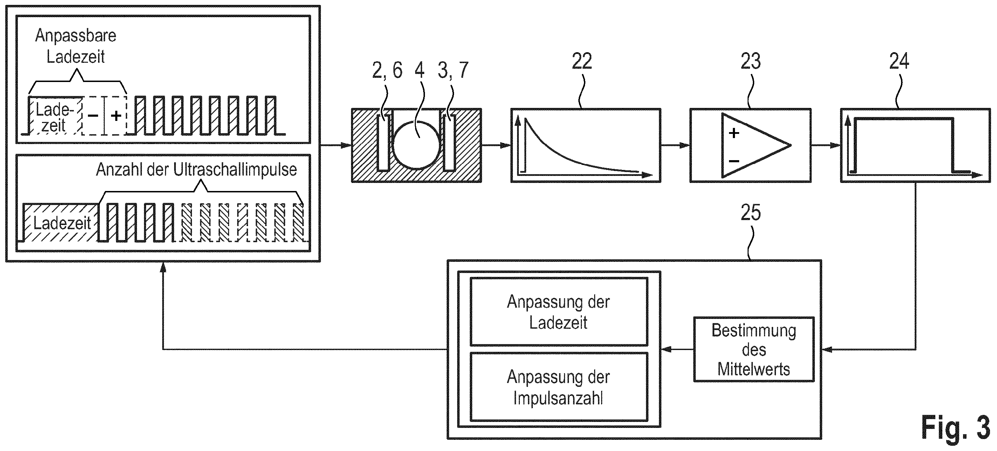

In Fig. 3 ist gezeigt, wie gemäß eines Ausführungsbeispiels der vorliegenden Erfindung die Aufladezeit des Ultraschallsenders 2 und/oder die Anzahl der Ultraschallimpulse angepasst werden, um die Menge der bei der Messung applizierten Ultraschallenergie zu variieren. Durch die kombinierte Anpassung von Ladezeit und Impulsanzahl kann der Energieinhalt der ausgesendeten Impulse präzise geregelt werden. Dabei arbeitet ein Algorithmus die in Fig. 3 gezeigten Schritte ab. Im ersten Fenster ist gezeigt, mittels welcher Parameter die Ultraschallenergie variiert werden kann. Dabei kann die Aufladezeit des Ultraschallsenders 2 und/oder die Anzahl der vom Ultraschallsender 2 emittierten Ultraschallimpulse variiert werden. Nach der Emission der Ultraschallimpulse in den Infusionsschlauch 4, empfängt der Ultraschallempfänger 3 Ultraschallsignale 22. Analog ist dies auch möglich mit dem Aufbau umfassend Ultraschallsende- und Empfangseinheit 6 sowie Ultraschallreflektor 7, wobei die Ultraschallimpulse von der Ultraschallsende- und Empfangseinheit 6 durch den Infusionsschlauch 4 zum Ultraschallreflektor 7 geleitet werden, welches die Impulse wieder durch den Infusionsschlauch 4 zur Ultraschallsende- und Empfangseinheit 6 leitet. Dabei liefern die einzelnen Ultraschallimpulse Ultraschallsignale 22, die typischerweise exponentiell mit der Zeit an Intensität verlieren. Im folgenden Schritt werden die Ultraschallsignale 22 durch einen Komparator 23, der Teil der Steuer- und Auswerteeinheit 8 ist, analysiert. Dabei wird das Zeitintervall ermittelt, in welchem das Ultraschallsignal 22 über der aktuell festgelegten Alarmschwelle liegt (Komparatorsignal 24). Aus mehreren Zeitintervallen wird dann der Durchschnitt oder eine andere statistische Größe errechnet, worauf basierend eine Steuer- und Auswerteeinheit 8 eine Anpassung der Anzahl der Ultraschallimpulse vornehmen kann, je nachdem ob sich der Durchschnitt oder eine andere statistische Größe innerhalb, über oder unterhalb eines vorher definierten Toleranzbereichs oder -werts befindet. In einer alternativen Ausführungsform kann die Bestimmung des Mittelwerts oder eine andere statistische Größe und die darauf basierende Anpassung der Aufladezeit des Ultraschallsenders bzw. der Zahl der pro Zeiteinheit emittierten Ultraschallimpulse in einer Mikrokontrolleinheit 25 erfolgen, die Teil einer Steuer- und Auswerteeinheit 8 ist.

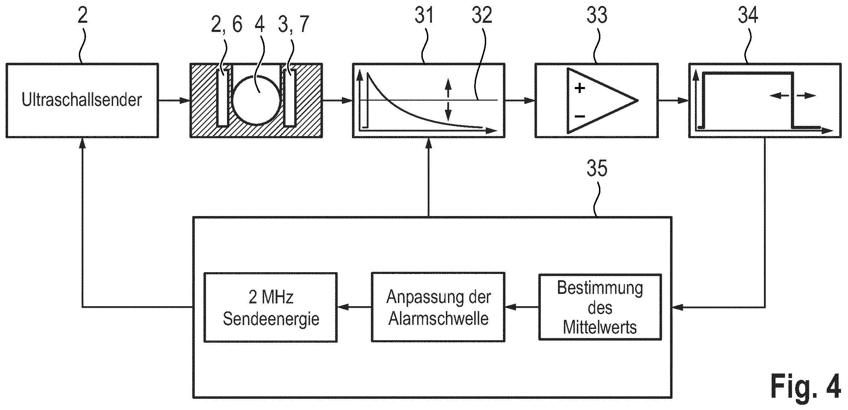

Alternativ kann gemäß eines weiteren vorteilhaften Ausführungsbeispiels der zugrundeliegenden Erfindung, gezeigt in Fig. 4, die Höhe der Alarmschwelle 32 angepasst werden. Dabei löst eine Steuer- und Auswerteeinheit 8 typischerweise einen Alarm aus, sobald ein zuvor festgelegter Grenzwert der positiven Pulsbreite des Komparatorsignals 34 unterschritten wird. Die positive Pulsbreite des Komparatorsignals 34 ergibt sich aus dem Zeitintervall, in dem das Ultraschallsignal 31 oberhalb der Alarmschwelle 32 liegt. Die Anpassung der Alarmschwelle 32 läuft dabei wie folgt ab: Der Ultraschallsender 2 sendet Ultraschallimpulse durch den Infusionsschlauch 4 zum Ultraschallempfänger 3. Analog ist dies auch möglich mit dem Aufbau umfassend Ultraschallsende- und Empfangseinheit 6 sowie Ultraschallreflektor 7, wobei die Ultraschallimpulse von der Ultraschallsende- und Empfangseinheit 6 durch den Infusionsschlauch 4 zum Ultraschallreflektor 7 geleitet werden, welches die Impulse wieder durch den Infusionsschlauch 4 zur Ultraschallsende- und Empfangseinheit 6 leitet. Dabei liefern die einzelnen Ultraschallimpulse Ultraschallsignale 31, die typischerweise exponentiell mit der Zeit an Intensität verlieren. Im folgenden Schritt werden die Ultraschallsignale 31 durch einen Komparator 33, der Teil der Steuer- und Auswerteeinheit 8 ist, analysiert. Dabei wird das Zeitintervall 34 ermittelt, in welchem das Ultraschallsignal 31 über des aktuell festgelegten Alarmschwelle 32 liegt. Aus mehreren Zeitintervallen wird dann der Durchschnitt oder eine andere statistische Größe errechnet, worauf basierend die Steuer- und Auswerteeinheit 8 eine Anpassung der Alarmschwelle 32 vornehmen kann, je nachdem ob sich der Durchschnitt oder eine andere statistische Größe innerhalb, über oder unterhalb eines vorher definierten Toleranzbereichs oder -werts befindet. In einer alternativen Ausführungsform kann die Bestimmung des Mittelwerts oder eine andere statistische Größe und die darauf basierende Anpassung der Alarmschwelle in einer Mikrokontrolleinheit 35 erfolgen, die Teil der Steuer- und Auswerteeinheit 8 ist.

In einer besonders bevorzugten Ausgestaltung können die beiden Varianten gemäß Fig. 3 und Fig. 4 miteinander in einer gemeinsamen Steuer- und Auswerteeinheit 8 kombiniert sein, und die beschriebenen Prozesse - Anpassung der Alarmschwelle und der Sendeenergie - können je nach Bedarf individuell oder zusammen (simultan) ablaufen.

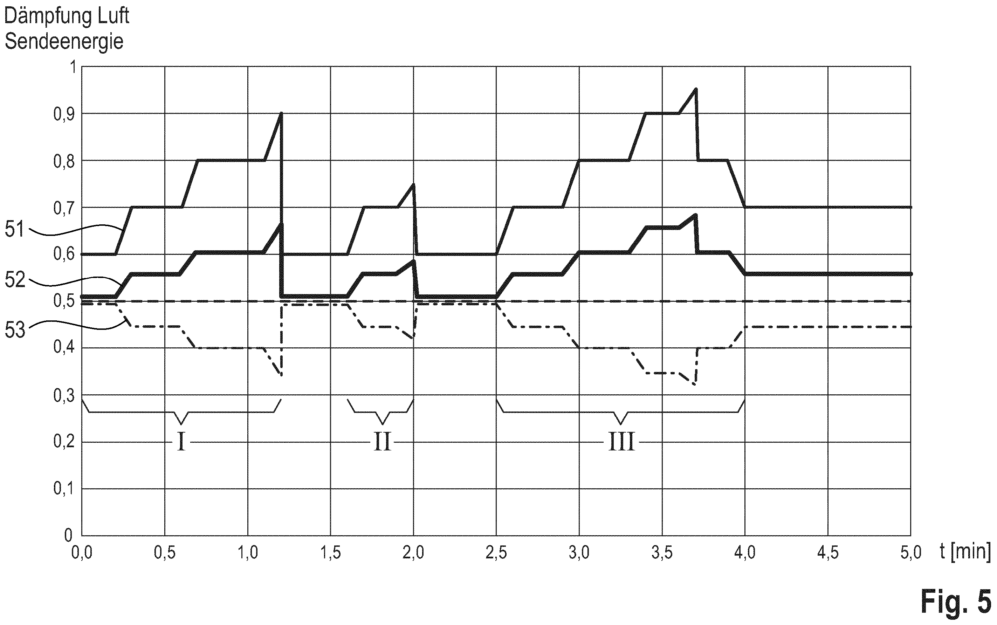

In Fig. 5 ist ein beispielhafter Verlauf der Dämpfung des Ultraschallsignals 51 über die Zeit gezeigt, welcher erfindungsgemäß durch die Steuer- und Auswerteeinheit 8 verarbeitet wird. Die Steuer- und Auswerteeinheit 8 regelt basierend auf der erfassten Dämpfung des Ultraschallsignals 51 die Alarmschwelle 53 und die Menge der eingekoppelten Ultraschallenergie 52. Dabei sammeln sich im vorliegenden Beispiel zunächst nacheinander drei stehende Luftblasen im Sensorbereich 5 des Infusionsschlauchs 4 an (Abschnitt I in Fig. 5), erkennbar durch drei Anstiege der Dämpfung des Ultraschallsignals 51 von zunächst 0,6 auf 0,9. Nach Erreichen dieses Werts fällt die Dämpfung der Ultraschallsignals 51 rapide auf einen Wert von 0,6, was zeigt, dass die zuvor angesammelten Luftblasen gesammelt den Sensorbereich 5 verlassen haben. Daraufhin kommt es zu zwei erneuten Anstiegen der Dämpfung des Ultraschallsignals, zuerst von 0,6 auf 0,7, kurz darauf gefolgt von 0,7 auf 0,75 (Abschnitt II in Fig. 5). Nach Erreichen des letzten Werts fällt die Dämpfung des Ultraschallsignals 51 rapide auf 0,6 ab. Dieser Verlauf repräsentiert das Ansammeln von zwei stehenden Luftblasen, von denen die zweite kleiner als die Erste ist, wobei während des Ansammelns der zweiten Luftblase sich die bisher im Sensorbereich 5 gesammelte Luftmenge löst und sich im Infusionsschlauch 4 fortbewegt. Daraufhin (Abschnitt III in Fig. 5) kommt es zu drei weiteren Anstiegen der Dämpfung des Ultraschallsignals 51 um jeweils 0,1 von 0,6 auf 0,9. Dies repräsentiert das Ansammeln von drei gleich großen Luftblasen im Sensorbereich 5. Nachdem sich weitere Luft im Sensorbereich 5 anlagert, gezeigt durch den Anstieg der Dämpfung des Ultraschallsignals 51 von 0,9 auf 0,95, fällt die Dämpfung zurück auf 0,8, was einer teilweisen Ablösung der bisher gesammelten Luftmenge im Sensorbereich 5 entspricht. Nach kurzer Zeit fällt die Dämpfung des Ultraschallsignals auf einen Wert von 0,7, was einer weiteren Entfernung von Luft aus dem Sensorbereich 5 entspricht. Daraufhin bleibt die Dämpfung des Ultraschallsignals 51 konstant. Im Vergleich zum niedrigsten Wert der Dämpfung des Ultraschallsignals 51 von 0,6 lässt sich ableiten, dass dabei eine Restmenge an Luft im Sensorbereich 5 verbleibt. Proportional zur Höhe der Dämpfung des Ultraschallsignals 51 erhöht dabei die Steuer- und Auswerteeinheit 8 die Menge der eingekoppelten Ultraschallenergie 52, um die Empfindlichkeit des Verfahrens bzw. des Gassensors 1 aufrechtzuerhalten. Analog dazu kann die Alarmschwelle 53 entsprechend abgesenkt werden, um das Systemverhalten für eine Detektion von Gasvolumen konstant zu halten.

In einem alternativen Beispiel ist anstelle eines Infusionsschlauchs 4 ein Blutschlauch 10 (z. B. als Bestandteil eines extrakorporalen Blutkreislaufs) eines Dialysesystems vorhanden.

Insgesamt erlaubt das beschriebene System eine adaptive Detektion, die Toleranzen ausgleichen kann. Der Sensor kann zwischen stehenden und bewegten Blasen unterscheiden und erkennt über Signalverläufe auch das Verlassen des Sensorbereichs durch einzelne Gasblasen. Dabei können sowohl deren Anzahl als auch deren ungefähres Volumen durch Auswertung der Signalverläufe bestimmt werden.

### Bezugszeichenliste

- 1: Gassensor
- 2: Ultraschallsender
- 3: Ultraschallempfänger
- 4: Infusionsschlauch
- 5: Detektionsbereich
- 6: Ultraschallsende- und Empfangseinheit
- 7: Ultraschallreflektor
- 8: Steuer- und Auswerteeinheit
- 10: Blutschlauch
- 22: Ultraschallsignal
- 23, 33: Komparator
- 24, 34: Komparatorsignal
- 25, 35: Mikrokontrolleinheit
- 32, 53: Alarmschwelle
- 51: Dämpfung des Ultraschallsignals
- 52: Eingekoppelte Ultraschallenergie

## Patentansprüche

1. Verfahren zur Detektion von Gasblasen, insbesondere Luftblasen, in einem von einem Infusionsmedium durchströmten Infusionsschlauch (4) oder in einem von Blut durchströmten Blutschlauch (10) eines Dialysesystems, bei dem ein ultraschallbasierter Gassensor (1) eingesetzt wird, umfassend die Schritte:
• Applizieren von Ultraschallimpulsen auf den Infusionsschlauch (4) oder Blutschlauch (10) mittels eines Ultraschallsenders (2),
• Detektion von durch den Infusionsschlauch (4) oder Blutschlauch (10) propagierten Ultraschallimpulsen durch einen Ultraschallempfänger (3),
• Auswertung eines vom Ultraschallempfänger (3) ausgegebenen Sensorsignals durch eine Steuer- und Auswerteeinheit (8),
• Vergleich des Sensorsignals oder eines daraus abgeleiteten Signals mit einem vorgegebenen Schwellenwert zur Erkennung von Gasblasen,
• Ausgabe eines Alarmsignals, wenn das Sensorsignal den Schwellenwert überschreitet oder unterschreitet,
wobei während der Auswertung mindestens einer der folgenden Parameter automatisiert angepasst oder variiert wird:
• die mittels der Ultraschallimpulse in den Infusionsschlauch (4) oder Blutschlauch (10) eingebrachte Sendeenergie,
• der Schwellwert zur Auslösung des Alarmsignals.

2. Verfahren nach Anspruch 1, wobei die Anpassung der Sendeenergie durch eine Steuerung der Anzahl aufeinanderfolgender Ultraschallimpulse und/oder durch Regelung des Energieinhalts einzelner Impulse erfolgt.

3. Verfahren nach einem der vorhergehenden Ansprüche, wobei der Schwellenwert zur Auslösung des Alarmsignals dynamisch in Abhängigkeit vom Verlauf des Sensorsignals über die Zeit angepasst wird.

4. Verfahren nach einem der vorhergehenden Ansprüche, wobei die Anpassung der Sendeenergie anhand eines vorgegebenen Zielbereichs für das Sensorsignal erfolgt, in dem anhand des Sensorsignals zuverlässig die Menge und das Volumen der Gasblasen im Infusionsschlauch (4) oder Blutschlauch (10) detektiert werden können, und wobei vorteilhafterweise die aktuelle Sensorsignalgröße kontinuierlich mit diesem Zielbereich verglichen wird.

5. Verfahren nach einem der vorhergehenden Ansprüche, wobei die automatisierte Anpassung durch einen in der Steuer- und Auswerteeinheit (8) implementierten Algorithmus erfolgt, der in der Lage ist, charakteristische Signalverläufe physikalischen Phänomenen im Infusionsschlauch (4) oder Blutschlauch (10), insbesondere stehenden oder bewegten Gasblasen, zuzuordnen.

6. Verfahren nach Anspruch 5, wobei der Algorithmus auf einer Lookup-Tabelle oder auf einem lernfähigen Modell basiert, das während des Betriebs oder im Vorfeld trainiert wurde.

7. Verfahren nach einem der vorhergehenden Ansprüche, wobei zusätzlich zur Detektion von Gasblasen deren zeitliche Bewegung oder Entfernung aus dem Sensorbereich erkannt wird und eine Bilanzierung des Gasvolumens auf Basis der Signalverläufe erfolgt.

8. Verfahren nach einem der vorhergehenden Ansprüche, wobei ein Alarm nur dann ausgelöst wird, wenn die Überschreitung des Schwellenwerts über eine vorgegebene Mindestzeit andauert oder eine bestimmte Flankensteilheit des Sensorsignals überschreitet.

9. Verfahren nach einem der vorhergehenden Ansprüche, wobei eine Plausibilitätsprüfung der Sensorsignale erfolgt, um zwischen Gasblasendetektion und Signalveränderungen aufgrund von Toleranzen oder äußeren Störeinflüssen zu unterscheiden.

10. Verfahren nach einem der vorhergehenden Ansprüche, wobei die Auswertung des Sensorsignals kontinuierlich oder in diskreten Intervallen erfolgt, abhängig von einem Betriebsmodus einer zugeordneten Infusionseinrichtung oder eines Dialysegeräts, insbesondere einem Vorbereitungsmodus oder Therapiebetrieb.

11. Verfahren nach einem der vorhergehenden Ansprüche, wobei die Steuer- und Auswerteeinheit (8) zusätzlich mit Informationen über das verwendete Infusionsmedium, die Flussrate oder die geometrische Position des Infusionsschlauchs (4) oder des Blutschlauchs (10) gespeist wird, und diese Informationen in die Anpassung der Parameter einbezogen werden.

12. Verfahren nach einem der vorhergehenden Ansprüche, wobei das Volumen einer im Infusionsschlauch (4) oder Blutschlauch (10) festsitzenden Gasblase bestimmt wird, wenn sich diese löst, indem ein Signalanstieg im Empfangssignal analysiert wird, wobei die Analyse bevorzugt durch eine automatische Anpassung der Sendeenergie und/oder der Alarmschwelle erfolgt, und das ermittelte Volumen der sich lösenden Gasblase in eine Gesamtbilanzierung von detektierten Gasblasenvolumina einfließt.

13. Vorrichtung zur Detektion von Gasblasen in einem von einem Infusionsmedium durchströmbaren Infusionsschlauch (4) oder in einem von Blut durchströmbaren Blutschlauch (10) eines Infusionssystems, umfassend:
• einen Ultraschallsender (2), der eingerichtet ist, Ultraschallimpulse auf den Infusionsschlauch (4) oder Blutschlauch (10) zu applizieren,
• einen Ultraschallempfänger (3), der eingerichtet ist, durch den Infusionsschlauch (4) oder Blutschlauch (10) propagierte Ultraschallimpulse zu detektieren,
• eine Steuer- und Auswerteeinheit (8), die mit dem Ultraschallempfänger (3) verbunden ist und eingerichtet ist, ein vom Ultraschallempfänger (3) erzeugtes Sensorsignal auszuwerten,
• eine Vergleichseinheit zur Bewertung des Sensorsignals oder eines davon abgeleiteten Signals im Hinblick auf einen vorgegebenen Schwellenwert zur Erkennung von Gasblasen,
• eine Alarmeinheit zur Ausgabe eines Alarmsignals, wenn das Sensorsignal den Schwellenwert über- oder unterschreitet,
wobei die Vorrichtung eine Steuer- und Auswerteeinheit (8) aufweist, die eingerichtet ist, während des Betriebs mindestens einen der folgenden Parameter automatisiert anzupassen:
• die durch die Ultraschallimpulse eingebrachte Sendeenergie durch Variation der Anzahl und/oder des Energieinhalts der Impulse,
• den Schwellenwert zur Auslösung des Alarmsignals.

14. Vorrichtung nach Anspruch 13, wobei die Steuer- und Auswerteeinheit (8) eingerichtet ist, die Sendeenergie anhand eines vorgegebenen Zielbereichs für das Sensorsignal zu regeln, insbesondere indem die aktuelle Signalgröße mit dem Zielbereich verglichen und die Anzahl und/oder der Energieinhalt der Ultraschallimpulse entsprechend angepasst wird.

15. Vorrichtung nach einem der Ansprüche 13 oder 14, wobei die Steuer- und Auswerteeinheit (8) einen Algorithmus implementiert, der eingerichtet ist, unterschiedliche Signalverläufe charakteristischen Vorkommnissen im Infusionsschlauch (4) oder Blutschlauch (10), insbesondere stehenden oder bewegten Gasblasen, zuzuordnen.
